(19) Europäisches Patentamt
European Patent Office
Office européen des brevets

(11) EP 2 730 212 A1

(12) **EUROPEAN PATENT APPLICATION**
published in accordance with Art. 153(4) EPC

(43) Date of publication:
**14.05.2014 Bulletin 2014/20**

(21) Application number: **13810578.8**

(22) Date of filing: **28.03.2013**

(51) Int Cl.:
***A61B 1/00*** (2006.01) ***G02B 23/26*** (2006.01)

(86) International application number:
**PCT/JP2013/059241**

(87) International publication number:
**WO 2014/002556 (03.01.2014 Gazette 2014/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **28.06.2012 JP 2012145926**

(71) Applicants:
- **Olympus Medical Systems Corp.**
  **Tokyo 151-0072 (JP)**
- **Olympus Corporation**
  **Shibuya-ku**
  **Tokyo 151-0072 (JP)**

(72) Inventors:
- **SAKAI, Yuji**
  **Tokyo 151-0072 (JP)**
- **FUNAKUBO, Tomoki**
  **Tokyo 151-0072 (JP)**
- **SHIMIZU, Morimichi**
  **Tokyo 151-0072 (JP)**

(74) Representative: **Gunzelmann, Rainer**
**Wuesthoff & Wuesthoff**
**Patent- und Rechtsanwälte**
**Schweigerstraße 2**
**81541 München (DE)**

# (54) SCANNING ENDOSCOPE AND SCANNING ENDOSCOPE MANUFACTURING METHOD

(57) A scanning endoscope 2 includes an optical fiber 14 that guides a light with which a living body is illuminated, a holding member 41 having an insertion hole 41h through which the optical fiber 14 is inserted, in which the optical fiber 14 is provided so as to extend from a distal end by a predetermined length and a concave portion 41g that communicates with the insertion hole 41h is formed on the distal end, a drive portion 15 that is provided in the holding member 41, and causes a free end of the optical fiber 14 that extends from the distal end of the holding member 41 to scan, and an adhesive portion 46 that is coated on or filled in the concave portion 4 1 g to firmly fix the optical fiber 14 and the holding member 41 to each other, and has a plane which is flush with the distal end of the holding member 41 formed thereon, whereby a variation in length of the free end of the optical fiber in each of respective individual pieces at a production time is eliminated, and a scanning characteristic of the optical fiber becomes constant in each of the respective individual pieces.

**FIG.17**

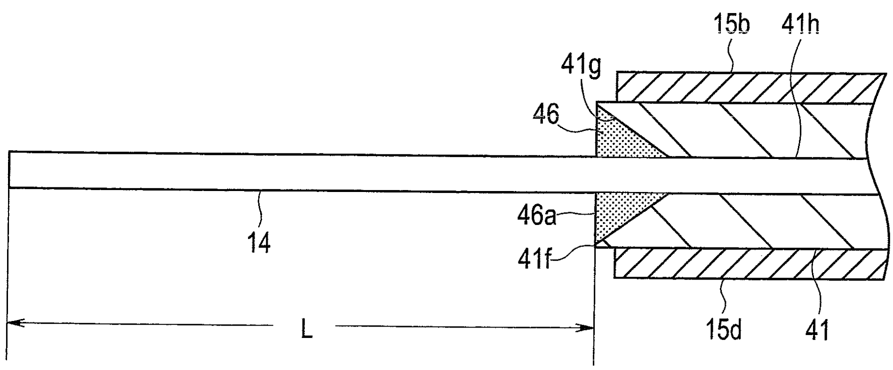


EP 2 730 212 A1

## Description

### Technical Field

The present invention relates to a scanning endoscope that detects a return light by causing illumination fibers that emit an illuminating light to scan, and converts the return light into an image, and a production method of the scanning endoscope.

### Background Art

As is well known, there is an electronic endoscope that photoelectrically converts a subject image by an image pickup apparatus having a solid image pickup device such as a CCD and a CMOS, and displays an acquired image on a monitor. In recent years, as an apparatus that performs image display of an object image without using the art of a solid image pickup device like this, a scanning endoscope apparatus has been known which causes distal ends of optical fibers for illumination that guide light from a light source to scan two-dimensionally, receives a return light from a subject with a fiber bundle for light reception, and makes a two-dimensional image by using light intensity signals that are sequentially detected with time.

The art of acquiring an image by causing optical fibers to scan as above is disclosed in, for example, an optical scanning fiber apparatus including an endoscope described in Japanese Patent Application Laid-Open Publication No. 2010-513949. In the conventional optical scanning fiber apparatus, optical fibers are mounted to an actuator tube body by being bonded to the actuator tube body like a cantilever beam in such a manner that one ends of the optical fibers become free ends. It is indicated that at this time, in the optical scanning fiber apparatus of Japanese Patent Application Laid-Open Publication No. 2010-513949, the optical fibers are desirably bonded in such a manner that the shape of the bonded portions of the optical fibers becomes the shape of a perfect circular cone.

Incidentally, in the conventional optical scanning fiber apparatus, an actuator is driven at a frequency in the vicinity of a resonant frequency in order to scan the optical fiber. Since the resonant frequency at this time is significantly dependent on a length of a free end in accordance with a diameter of the optical fiber, in order to enhance reproductivity of the resonant frequency of the actuator, it is necessary to keep the length of the free end of the optical fiber, and precision is required in fixing the optical fiber to a fiber holding member provided with the actuator.

However, it is very difficult to apply an extremely small amount of adhesive to precisely form a perfect cone in a situation where it is required to perform a work of causing the optical fiber which tends to be broken by external force to adhere to the fiber holding member without causing damage, as performed in the optical scanning fiber apparatus of Japanese Patent Application Laid-Open Publication No. 2010-513949. Therefore, in the conventional optical scanning fiber apparatus, the length of the free end of the optical fiber is not made constant to cause a manufacturing individual difference of an optical scanning unit and cause dispersion in scanning characteristics of the optical fiber.

Therefore, the present invention has been made in view of the foregoing circumstances and an object of the present invention is to provide a scanning endoscope and a production method of the scanning endoscope which eliminate dispersion in length of free ends of individual optical fibers in manufacture to make scanning characteristics of the individual optical fibers constant.

### Disclosure of Invention

### Means for Solving the Problem

In order to achieve the above object, a scanning endoscope of one aspect of the present invention includes an optical fiber that guides a light with which a living body is illuminated, a holding member having an insertion hole through which the optical fiber is inserted, in which the optical fiber is provided so as to extend from a distal end by a predetermined length and a concave portion that communicates with the insertion hole is formed at the distal end, a drive portion that is provided in the holding member, and causes a free end of the optical fiber that extends from the distal end of the holding member to scan, and an adhesive portion that is coated on or filled in the concave portion to firmly fix the optical fiber and the holding member to each other, and has a plane which is flush with the distal end of the holding member formed thereon.

Furthermore, a production method of a scanning endoscope of one aspect of the present invention includes: in a production method of the scanning endoscope including an optical fiber that guides a light with which a living body is illuminated, a holding member having an insertion hole through which the optical fiber is inserted, in which the optical fiber is provided so as to extend from a distal end by a predetermined length and a concave portion that communicates with the insertion hole is formed at the distal end, a drive portion that is provided in the holding member, and causes a free end of the optical fiber that extends from the distal end of the holding member to scan, and an adhesive portion that is coated on or filled in the concave portion to firmly fix the optical fiber and the holding member to each other, and has a plane which is flush with the distal end of the holding member formed thereon, introducing the optical fiber into the insertion hole of the holding member; extending the optical fiber to have a predetermined length from the distal end of the holding member, coating or filling the concave portion with an adhesive, scraping off an excess of the adhesive in accordance with a distal end face of the holding member to form a plane, and curing the adhesive to form the adhesive portion.

Brief Description of the Drawings

Fig. 1 is a view showing a configuration of a scanning endoscope apparatus having a scanning endoscope of a first embodiment;
Fig. 2 is a sectional view showing a configuration of a light scanning unit of the first embodiment;
Fig. 3 is a sectional view taken along the III-III line of Fig. 2 of the first embodiment;
Fig. 4 is a sectional view of an actuator of a scanning endoscope that is a sectional view of an actuator of a modification of the first embodiment;
Fig. 5 is a diagram for explaining an example of a signal waveform that is supplied to an actuator of the first embodiment;
Fig. 6 is a view for explaining an example of a scanning locus of an illumination fiber of the first embodiment;
Fig. 7 is a graph showing a relation of a length of an illumination fiber free end of a fiber diameter of 125 μm and a primary resonance frequency of the first embodiment;
Fig. 8 is a graph showing a vibration characteristic of the illumination fiber showing a relation of a drive frequency and an amplitude of the first embodiment;
Fig. 9 is a perspective view showing the configuration of the light scanning unit of the first embodiment;
Fig. 10 is a perspective view showing a configuration of a concave portion that is formed in a ferrule of the first embodiment;
Fig. 11 is a front view showing the configuration of the concave portion that is formed in the ferrule of the first embodiment;
Fig. 12 is a sectional view showing the configuration of the concave portion that is formed in the ferrule of the first embodiment;
Fig. 13 is a perspective view showing a configuration of a concave portion that is formed in a ferrule of a modification of the first embodiment;
Fig. 14 is a front view showing the configuration of the concave portion that is formed in the ferrule of the modification of the first embodiment;
Fig. 15 is a sectional view showing a production process of inserting the illumination fiber through the ferrule of the first embodiment;
Fig. 16 is a sectional view showing a production process in which the concave portion of the ferrule is coated or filled with an adhesive of the first embodiment;
Fig. 17 is a sectional view showing a production process in which an excess of the adhesive is scraped off in accordance with a distal end of the ferrule of the first embodiment;
Fig. 18 is a sectional view showing a production process in which the adhesive is in a sunken state by curing of the first embodiment;
Fig. 19 is a sectional view showing a production process in which the sunken portion is coated or filled with the adhesive again of the first embodiment;
Fig. 20 is a sectional view showing a production process in which an excess of the adhesive is scraped off in accordance with the distal end of the ferrule of the first embodiment;
Fig. 21 is an exploded perspective view showing a configuration of a light scanning unit of a second embodiment;
Fig. 22 is a perspective view showing a configuration of the light scanning unit of the second embodiment;
Fig. 23 is an exploded perspective view showing a configuration of a light scanning unit of a modification of the second embodiment; and
Fig. 24 is a perspective view showing the configuration of the light scanning unit of the modification of the second embodiment.

Best Mode for Carrying Out the Invention

Hereinafter, an endoscope that is the present invention will be described. Note that in the following explanation, the drawings based on respective embodiments are schematic, and attention should be paid to the fact that relations of thicknesses and widths of respective parts, ratios of thicknesses of the respective parts and the like differ from actual ones. Parts in which dimensional relations and ratios differ from one another are also included among the drawings in some cases.

(First Embodiment)

First, with use of Fig. 1 to Fig. 20, a configuration of a scanning endoscope apparatus having a scanning endoscope of a first embodiment of the present invention will be described hereinafter. Fig. 1 is a view showing a configuration of a scanning endoscope apparatus having a scanning endoscope. Fig. 2 is a sectional view showing a configuration of a light scanning unit. Fig. 3 is a sectional view taken along the III-III line of Fig. 2. Fig. 4 is a sectional view of an actuator of a scanning endoscope that is a sectional view of an actuator of a modification. Fig. 5 is a diagram for explaining an example of a signal waveform that is supplied to the actuator. Fig. 6 is a view for explaining an example of a scanning locus of an illumination fiber. Fig. 7 is a graph showing a relation of a length of an illumination fiber free end of a fiber diameter of 125 μm and a primary resonance frequency. Fig. 8 is a graph showing a vibration characteristic of the illumination fiber showing a relation of a drive frequency and an amplitude. Fig. 9 is a perspective view showing the configuration of the light scanning unit. Fig. 10 is a perspective view showing a configuration of a concave portion that is formed in a ferrule. Fig. 11 is a front view showing the configuration of the concave portion that is formed in the ferrule. Fig. 12 is a sectional view showing the configuration of the concave portion that is formed in the ferrule. Fig. 13 is a perspective view showing a configuration of a concave portion that is formed in a ferrule of a modification. Fig. 14 is a front view showing the configuration of the concave portion that is formed in the ferrule of the modification. Fig. 15 is a sectional view showing a production process of inserting the illumination fiber through the ferrule. Fig. 16 is a sectional view showing a production process in which the concave portion of the ferrule is coated or filled with an adhesive. Fig. 17 is a sectional view showing a production process in which an excess of the adhesive is scraped off in accordance with a distal end of the ferrule. Fig. 18 is a sectional view showing a production process in which the adhesive is in a sunken state by curing. Fig. 19 is a sectional view showing a production process in which the shrunk portion is coated or filled with the adhesive again. Fig. 20 is a sectional view showing a production process in which an excess of the adhesive is scraped off in accordance with the distal end of the ferrule.

As shown in Fig. 1, a scanning endoscope apparatus (hereinafter, simply called an endoscope apparatus) 1 is configured by having a scanning endoscope (hereinafter, simply called an endoscope) 2 that irradiates a subject with an illuminating light while causing the illuminating light to scan, and obtains a return light from the subject, a main body apparatus 3 that is connected to the endoscope 2, and a monitor 4 that displays a subject image obtained in the main body apparatus 3.

The endoscope 2 is configured with a tube body including predetermined flexibility as a main body, and has an elongated insertion portion 11 that is inserted through an inside of a living body. At a distal end side of the insertion portion 11, a distal end portion 12 is provided. Furthermore, at a proximal end side of the insertion portion 11, a connector and the like not illustrated are provided, and the endoscope 2 is configured to be attachable to and detachable from the main body apparatus 3 via the connector and the like.

A distal end face 12a of the distal end portion 12 is provided with an illumination optical system 13 configured by illumination lenses 13a and 13b, and a detection optical system 16a. Furthermore, in an inside of the insertion portion 11, a light scanning unit 40 is loaded which is provided with the illumination optical system 13, an illumination fiber 14 that is an optical fiber as an optical element that is inserted from a proximal end side to a distal end side, guides a light from a light source unit 24, which will be described later, and irradiates a living body with an illuminating light, and an actuator 15 that is provided at a distal end side of the illumination fiber 14, and causes a distal end of the illumination fiber 14 to scan in a desired direction based on a drive signal from a driver unit 25, which will be described later. With the configuration as above, an object is irradiated with the illuminating light from the light source unit 24, which is guided by the illumination fiber 14 of the light scanning unit 40.

Furthermore, in the inside of the insertion portion 11, a detection fiber 16 as a light receiving portion that is inserted from the proximal end side to the distal end side along an inner circumference of the insertion portion 11, and receives a return light from a subject is provided. At a distal end of the detection fiber 16, the detection optical system 16a described above is placed.

Note that the detection fiber 16 may be at least two or more fiber bundles. When the endoscope 2 is connected to the main body apparatus 3, the detection fiber 16 is connected to a demultiplexer 36, which will be described later.

Furthermore, in the inside of the insertion portion 11, a memory 17 that stores various kinds of information relating to the endoscope 2 is provided. The memory 17 is connected to a controller 23, which will be described later, via a signal line not illustrated when the endoscope 2 is connected to the main body apparatus 3, and various kinds of information relating to the endoscope 2 are read by the controller 23.

The main body apparatus 3 is configured by having a power supply 21, a memory 22, the controller 23, the light source unit 24, the driver unit 25, and a detection unit 26. The light source unit 24 is configured by having three light sources 31a, 31b and 31c and a multiplexer 32.

The driver unit 25 is configured by having a signal generator 33, a digital/analog (hereinafter, called D/A) converters 34a and 34b, and an amplifier 35.

The detection unit 26 is configured by having the demultiplexer 36, detectors 37a to 37c and analog/digital (hereinafter, called A/D) converters 38a to 38c. The power supply 21 controls supply of power to the controller 23 in response to an operation of a power supply switch or the like not illustrated. In the memory 22, a control program and the like for performing control of the entire main body apparatus 3 are stored.

When the controller 23 is supplied with power from the power supply 21, the controller 23 reads the control program from the memory 22, performs control of the light source unit 24 and the driver unit 25, performs analysis of a light intensity of a return light from an object that is detected in the detection unit 26, and performs control of subjecting a periphery of an obtained object image to masking processing as an image with a predetermined aspect ratio and causing the monitor 4 to display the image.

The light sources 31a, 31b and 31c of the light source unit 24 respectively emit lights of different wavelength bands, for example, lights of wavelength bands of R (red), G (green) and B (blue) to the multiplexer 32 based on control of the controller 23. The multiplexer 32 multiplexes the lights of the wavelength bands of R, G and B that are emitted from the light sources 31a, 31b and 31c, and emits the lights to the illumination fiber 14.

The signal generator 33 of the driver unit 25 outputs a drive signal for causing the distal end of the illumination fiber 14 to scan in a desired direction, for example, in an elliptic helical shape, based on the control of the controller 23. More specifically, the signal generator 33 outputs a drive signal that causes the distal end of the illumination fiber 14 to drive in a lateral direction (an X axis direction) with respect to an insertion axis of the insertion portion 11 to the D/A converter 34a, and outputs a drive signal that causes the distal end of the illumination fiber 14 to drive in a vertical direction (a Y axis direction) with respect to the insertion axis of the insertion portion 11 to the D/A converter 34b.

The D/A converters 34a and 34b convert the drive signals, which are respectively inputted, into analog signals from digital signals and outputs the analog signals to the amplifier 35. The amplifier 35 amplifies the inputted drive signals and outputs the drive signals to the actuator 15. The actuator 15 as a vibration portion causes the distal end (the free end) of the illumination fiber 14 to swing, and scan in the elliptic helical shape based on the drive signals from the amplifier 35. Thereby, the light emitted to the illumination fiber 14 from the light source unit 24 is sequentially emitted to the subject in the elliptic helical shape.

The detection fiber 16 receives a return light reflected on a surface region of a subject, and guides the received return light to the demultiplexer 36. The demultiplexer 36 is, for example, a dichroic mirror or the like, and demultiplexes the return light in a predetermined wavelength band. More specifically, the demultiplexer 36 demultiplexes the return light that is guided by the detection fiber 16 to return lights of the wavelength bands of R, G and B, and outputs the return lights to the detectors 37a, 37b and 37c, respectively.

The detectors 37a, 37b and 37c respectively detect light intensities of the return lights of the wavelength bands of R, G and B. Signals of the light intensities detected by the detectors 37a, 37b and 37c are respectively outputted to the A/D converters 38a, 38b and 38c. The A/D converters 38a to 38c convert the signals of the light intensities respectively outputted from the detectors 37a to 37c into digital signals from analog signals, and output the digital signals to the controller 23.

The controller 23 applies predetermined image processing to the digital signals from the A/D converters 38a to 38c to generate an object image, and displays the object image on the monitor 4.

Here, a detailed configuration of the light scanning unit 40 provided in the inside of the distal end portion 12 of the insertion portion 11 will be described with use of Fig. 2.

As shown in Fig. 2, the light scanning unit 40 is configured by having the illumination optical system 13 configured by the illumination lenses 13a and 13b, a barrel body 43 that holds the illumination optical system 13, a ferrule 41 as a holding member in which the illumination fiber 14 is inserted and disposed, and the actuator 15 is provided, and a holder 44 that holds the ferrule 41 together with the actuator 15 in the barrel body 43. Note that from the actuator 15, a lead wire 45 that is provided so as to extend from the driver unit 25 (see Fig. 1) is connected. Note that in the holder 44, a proximal end portion of the ferrule 41 is fitted so that the barrel body 43 which holds the illumination optical system 13 that is a distal end optical system, and the ferrule 41 are integrated. Moreover, in the holder 44, hole portions through which the illumination fiber 14 and a plurality of lead wires 45 are inserted are formed.

Furthermore, in more detail, between the illumination fiber 14 and the actuator 15, the ferrule 41 as a joining member is disposed as shown in Fig. 3. The ferrule 41 is a member that is used in the field of optical communications, zirconia (ceramics), nickel or the like is used for a material thereof, and center hole machining with high precision (for example, $\pm 1$ $\mu$m) with respect to an outside diameter (for example, 125 $\mu$m) of the illumination fiber 14 can be easily realized.

The ferrule 41 in this case is a quadrangular prism, and has side surfaces 42a and 42c perpendicular to the X axis direction, and side surfaces 42b and 42d perpendicular to the Y axis direction. Note that the ferrule 41 is not limited to a quadrangular prism, and can be a prism in any shape. To a substantial center of the ferrule 41, center hole machining based on the diameter of the illumination fiber 14 is applied, and the illumination fiber 14 is fixed by an adhesive or the like. For the center hole machining, a clearance (a gap) is made as small as possible, and an adhesive layer is made as thin as possible. Furthermore, for the adhesive, an adhesive with a low viscosity is used.

The actuator 15 is configured by four actuators 15a to 15d in this case, and the respective actuators 15a to 15d are provided at positions that are adjacent to the respective side surfaces 42a to 42d of the ferrule 41 of a quadrangular prism and are respectively point symmetric at 90°. The actuators 15a to 15d each have a configuration in which electrodes are provided on two distant surfaces of a piezoelectric element (a piezo element), for example, and expand and contract in response to the drive signals from the driver unit 25.

In particular, the two actuators 15a and 15c as a first drive portion drive in response to the drive signal from the D/A converter 34a, and the other two actuators 15b and 15d as a second drive portion drive in response to the drive signal from the D/A converter 34b. Thereby, the respective actuators 15a to 15d give vibration to the ferrule 41, cause the distal end of the illumination fiber 14 to swing, and cause the distal end of the illumination fiber 14 to scan in an elliptic helical shape. Note that the respective actuators 15a to 15d are not limited to piezoelectric transducers each configured by a piezoelectric element having a pair of electrodes, but may be, for example, coil-type transducers that are electromagnetically driven.

As a GND electrode for the respective actuators 15a to 15d, the ferrule 41 itself is used as the GND electrode when a conductive material such as nickel is used for the ferrule 41. Furthermore, as the GND electrode for the respective actuators 15a to 15d, conductive film work is applied onto a surface of the ferrule 41, which is used as the GND electrode, when a non-conductive material such as zirconia is used for the ferrule 41.

As above, in the endoscope 2, the ferrule 41 that is a joining member in which highly precise center hole machining is applied is inserted between the actuator 15 and the illumination fiber 14, whereby the adhesive layer required for fixation of the illumination fiber 14 and the ferrule 41 is made as thin as possible, an influence of a temperature change is reduced as much as possible, and stable drive of the illumination fiber 14 is realized.

Note that as shown in Fig. 4, the actuator 15 may be in a cylindrical shape. When the actuator 15 is formed into a cylindrical shape like this, the illumination fiber 14 is inserted and disposed in a center of a section thereof. An adhesive 47 is filled between the actuator 15 surrounding the illumination fiber 14 and the illumination fiber 14.

Note that the actuator 15 is provided with an electrode 48 on an inner circumferential face thereof, and is provided with four electrodes 49a to 49d on an outer circumferential face thereof. The four electrodes 49a to 49d are disposed with predetermined spaces provided from one another.

An operation of the endoscope apparatus 1 configured as above will be described hereinafter based on Fig. 5 and Fig. 6.

Note that Fig. 5(a) is a signal waveform of the drive signal that is outputted from the D/A converter 34a via the amplifier 35. The signal waveform is of the drive signal for causing the illumination fiber 14 to drive in the X axis direction, and is supplied to the actuators 15a and 15c.

Further, Fig. 5(b) is a signal waveform of the drive signal that is outputted from the D/A converter 34b via the amplifier 35. The signal waveform is of the drive signal for causing the illumination fiber 14 to drive in the Y axis direction, and is supplied to the actuators 15b and 15d.

The signal waveform in the Y axis direction is a signal waveform obtained by a phase of the signal waveform in the X axis direction shifted by 90°. More specifically, a phase difference between the signal waveform in the X axis direction and the signal waveform in the Y axis direction is calculated from the following (Equation 1) when the number N of vibration axes is an even number, and from the following (Equation 2) when the number N of vibration axes is an odd number.

$$\text{Phase difference} = 360° / (2 \times \text{number N of vibration axes}) \ldots \text{(Equation 1)}$$

$$\text{Phase difference} = 360° / \text{number N of vibration axes} \ldots \text{(Equation 2)}$$

In the present embodiment, the number N of vibration axes is 2 (an even number: the X axis and the Y axis), and therefore, the phase difference is 90° from the above described (Equation 1).

As above, the driver unit 25 configures a control section that generates a first drive signal that is outputted to the actuators 15a and 15c, and a second drive signal that is outputted to the actuators 15b and 15d, and controls a phase difference between a phase of the first drive signal and a phase of the second drive signal based on the number N of vibration axes.

As shown in Figs. 5(a) and (b), in the signal waveform, an amplitude becomes gradually larger from a time T1 to a time T2, and reaches the maximum amplitude value at the time T2. Subsequently, in the signal waveform, the amplitude becomes gradually smaller from the time T2 to a time T3, and reaches the minimum amplitude value at the time T3.

A scanning locus of the illumination fiber 14 at this time is a locus shown in Fig. 6. The distal end of the illumination fiber 14 is located at a position of an intersection point O of the X axis and the Y axis at the time T1. Subsequently, when the amplitude of the signal waveform becomes large from the time T1 to the time T2, the distal end of the illumination fiber 14 is caused to scan in a helical shape to an outer side from the intersection point O, and is located at a position of an intersection point Y1 with the Y axis, for example, at the time T2. Furthermore, when the amplitude of the signal waveform becomes small from the time T2 to the time T3, the distal end of the illumination fiber 14 is caused to scan in a helical shape to an inner side from the intersection point Y1, and is located at the position of the intersection point O at the time T3, though not illustrated.

As described as above, in the endoscope 2, the ferrule 41 that is the joining member in which highly precise center hole machining is applied is inserted between the actuator 15 and the illumination fiber 14. Thereby, the adhesive layer necessary for fixation of the illumination fiber 14 and the ferrule 41 is made thin, and the influence of a temperature change is reduced as much as possible. Accordingly, the endoscope is configured to reduce the influence of a temperature change, and to be able to perform stable drive of the illumination fiber without feedback control.

Incidentally, in the light scanning unit 40, in order to cause the illumination fiber 14 to scan efficiently, the number of drive vibrations of the actuator 15 is set so that the illumination fiber 14 drives at a predetermined resonance frequency. In the illumination fiber 14, the resonance frequency at which the illumination fiber 14 vibrates (swings) by a large amount is determined in accordance with the fiber diameter and the length of the free end that is a protruding amount (an extending amount) from the ferrule 41.

More specifically, in the light scanning unit 40, a primary resonance frequency (drive frequency) for obtaining the maximum amplitude with respect to the length of the free end (the extending amount from the ferrule 41) of the illumination fiber 14 with a fiber diameter (outside diameter) of, for example, 125 $\mu$m (hereinafter, the mention of the fiber diameter of 125 $\mu$m will be omitted in some cases) is set (assumed) from the theoretical value shown in the graph of Fig. 7. For example, the light scanning unit 40 is configured to obtain a vibration characteristic of the maximum amplitude of a3.5max as shown in the waveform of Fig. 8 by setting the drive frequency of the actuator 15 at approximately 8.0 KHz when the length of the free end of the illumination fiber 14 is set at 3.5 mm.

Here, in a production process of the light scanning unit 40, when the illumination fiber 14 is assembled to and firmly fixed to the ferrule 41, if an excessive adhesion or the like remains at the distal end of the ferrule 41, and the length of the free end of the illumination fiber 14 becomes 3.4 mm that is shorter than 3.5 mm by 0.1 mm, the primary resonance frequency (the drive frequency) for obtaining the maximum amplitude becomes approximately 8.5 KHz from the theoretical value shown in the graph of Fig. 7, and the actuator 15 is desirably driven so that the vibration characteristic of the maximum amplitude of a3.4max as shown in the waveform of Fig. 8 is obtained.

However, in the light scanning unit 40, the length of the free end of the illumination fiber 14 is set (assumed) at 3.5 mm and the drive frequency of the actuator 15 is set at 8.0 KHz. Therefore, if in the production process, the length of the free end of the illumination fiber 14 becomes short to be 3.4 mm due to occurrence of an error of -0.1 mm with respect to 3. 5 mm that is set, for example, the illumination fiber 14 does not vibrate by a large amount because the primary resonance frequency (drive frequency) for obtaining the maximum amplitude of a3.4 max is approximately 8.5 KHz.

Namely, if a manufacturing error of 0.1 mm between 3.5 mm and 3.4 mm of the length of the free end occurs (becomes short in this case) in the illumination fiber 14 of the fiber diameter of 125 $\mu$m, the primary resonance frequency (the drive frequency) at which the maximum amplitude is obtained changes by about 1.0 KHz, and a very large variation in the vibration characteristic occurs among the products. Namely, in the illumination fiber 14 with the fiber diameter of 125 $\mu$m, the amplitude changes in accordance with the waveform of the vibration characteristic of a full width at half maximum of about 0.1 KHz as shown in Fig. 8, and therefore, if the vibration characteristic for achieving the maximum amplitude shifts by as much as about 1.0 KHz by the length of the free end becoming shorter by 0.1 mm to 3.4 mm from 3.5 mm, the illumination fiber 14 hardly vibrates and cannot drive with the predetermined helical scanning, at the drive frequency of 8.0 KHz that is set (assumed).

Therefore, the light scanning unit 40 of the present embodiment is configured such that a concave portion in which the adhesive is filled is formed at the distal end of the ferrule 41, and the illumination fiber 14 is firmly fixed thereto.

More specifically, as shown in Fig. 9, the illumination fiber 14 is firmly fixed to the ferrule 41 of the light scanning unit 40 by an adhesive 46 as an adhesive portion having a surface portion 46a the surface position of which corresponds to a distal end face 41f. As shown in Fig. 10 to Fig. 12, in the ferrule 41 in this case, a conical (cone-shaped) concave portion 41g is formed, which is narrowed toward a proximal end side as an internal direction from the distal end face 41 f, and communicates with a fiber insertion hole 41h that is made by center hole machining. Namely, the concave portion 41g is in a conical shape that is formed to open on the distal end face 41 f of the ferrule 41, and to be narrowed toward the proximal end side.

In the ferrule 41, the illumination fiber 14 is inserted through and disposed in the fiber insertion hole 4 1 h to have a predetermined extending amount (the free end of a predetermined length) from the distal end face 41 f, and the concave portion 41 g is coated or filled with the adhesive 46 in such a manner that an intermediate outer circumferential portion of the illumination fiber 14 is covered with the adhesive 46 to be subjected to curing treatment. The adhesive 46 has the surface portion 46a formed to be flush with the distal end face 41f of the ferrule 41, and firmly fixes the illumination fiber 14 and the ferrule 41.

Note that the concave portion 4 1 g that is formed on the distal end face 41f of the ferrule 41 is formed into a conical shape formed to be narrowed toward the proximal end side, whereby the shape of the concave portion 41 g is preferable as the shape in which coating or filling of the adhesive 46 is easily performed without a gap, but the shape of the concave portion 41 g is not limited thereto, and may be formed into a pyramidal shape formed to be narrowed toward the proximal end side as shown in Fig. 13 and 14, for example, and besides, may be in any concave portion shape such as a spherical segment and a rectangular shape.

Here, a method (a production method) for assembling the illumination fiber 14 to the ferrule 41 will be described in detail hereinafter based on Fig. 15 to Fig. 20.

First, an assembly worker introduces the illumination fiber 14 into the fiber insertion hole 41h from the proximal end of the ferrule 41, and guides the illumination fiber 14 out from the distal end of the ferrule 41, as shown in Fig. 15. At this time, the assembly worker feeds out the illumination fiber 14 to a position where the illumination fiber 14 has a set predetermined length L (see Fig. 16) from the distal end face 41f of the ferrule 41 as a specified extending amount by using a micrometer or the like.

Next, the assembly worker coats or fills the concave portion 41g of the ferrule 41 with the adhesive 46 without a gap to cover a periphery of the illumination fiber 14, as shown in Fig. 16. Note that here, as the adhesive 46, for example, a thermosetting adhesive is used.

Subsequently, as shown in Fig. 17, the assembly worker scrapes off (levels off) an excessive portion of the adhesive 46 with a spatula or the like so that a plane which is flush with the distal end face 41 f of the ferrule 41 is formed to form the surface portion 46a as the plane, and performs thermal curing treatment to form the adhesive portion (46).

Note that when sinking occurs to the surface side of a cured adhesive 46b as shown in Fig. 18, the assembly worker coats or fills the sunk surface portion with an adhesive 46c again, as shown in Fig. 19. Subsequently, as shown in Fig. 20, the assembly worker scrapes off the excessive portion of the adhesive 46c with a spatula or the like so that the plane which is flush with the distal end face 41f of the ferrule 41 is formed again to form the surface portion 46a and performs thermal curing treatment. Note that the assembly worker repeatedly performs coating or filling of the adhesive 46 and thermal curing treatment repeatedly until the surface portion 46a of the adhesive 46 is formed to be flush with the distal end face 41f of the ferrule 41.

Here, for the adhesive 46 as the adhesive portion, a thermosetting adhesive is used, but the adhesive is not limited thereto, and an ultraviolet curable adhesive may be used. An ultraviolet curable adhesive is adopted as the adhesive 46 like this, whereby the advantage is provided that coating or filling of the adhesive 46 and ultraviolet curing treatment do not have to be repeatedly performed many times because sinking after curing hardly occurs, as compared with a thermosetting adhesive.

From the above, the concave portion 41 g is formed at the distal end of the ferrule 41, the concave portion 41g is coated or filled with the adhesive 46 that firmly fixes the illumination fiber 14 and the ferrule 41, and the surface portion 46a of the adhesive 46 is cured and formed so as to be the plane that is flush with the surface position of the distal end face 41f of the ferrule 41, whereby the light scanning unit 40 of the present embodiment can be produced so that an error does not occur to the length L of the free end of the illumination fiber 14 in each of the respective individual pieces.

By the above explanation, the scanning endoscope 2 of the present embodiment can be produced so that the scanning characteristic of the illumination fiber is constant in each of the respective individual pieces by including the light scanning unit 40 in which a variation in the length L of the free end of the illumination fiber 14 in each of the respective individual pieces at the production time is eliminated.

Note that in the above explanation, the mode of the ferrule 41 being a rectangular prism is cited as an example, but the present invention is not limited thereto, and for example, the concave portion 41 g which is coated or filled with the adhesive 46 may be formed on the distal end face of the ferrule 41 in the columnar shape shown in Fig. 4. The present invention is an art applicable to any shape of the ferrule 41 as a matter of course.

(Second Embodiment)

Next, a scanning endoscope apparatus of a second embodiment of the present invention will be described hereinafter with use of Fig. 21 to Fig. 24. Fig. 21 is an exploded perspective view showing a configuration of a light scanning unit. Fig. 22 is a perspective view showing the configuration of the light scanning unit. Fig. 23 is an exploded perspective view showing a configuration of a light scanning unit of a modification. Fig. 24 is a perspective view showing the configuration of the light scanning unit of the modification. Note that the configuration of the light scanning unit 40 in the scanning endoscope apparatus 1 in this case is a modification of the first embodiment, therefore the components already described will be assigned with the same reference signs, and explanation thereof will be omitted.

As shown in Fig. 21, the light scanning unit 40 in this case is configured such that the illumination fiber 14 is assembled and firmly fixed to the ferrule 41 by a block body 50 that is a fixing member in a conical shape that is the substantially same shape as the shape of the concave portion 41 g that is formed on the distal end face 41 f of the ferrule 41.

The block body 50 has, in a center, a hole portion through which the illumination fiber 14 is inserted, and fixes the illumination fiber 14 and the ferrule 41 by being fitted in the concave portion 41 g of the ferrule 41 by an adhesive. Furthermore, as shown in Fig. 22, in a state in which the block body 50 is firmly fixed to the concave portion 41 g of the ferrule 41, a surface portion 50a is a plane which is flush with the distal end face 41 f of the ferrule 41.

As above, the block body 50 is fitted in the concave portion 41g at the distal end of the ferrule 41 and is firmly fixed so that the surface portion 50a of the block body 50 becomes the plane which is flush with the surface position of the distal end face 41f of the ferrule 41, whereby the light scanning unit 40 of the present embodiment can also be produced so that an error does not occur to the length L of the free end of the illumination fiber 14 in each of the respective individual pieces.

Note that as shown in Fig. 23 and Fig. 24, the light scanning unit 40 may adopt a block body 51 in a shape having a distal end portion 52 in a conical shape in this case, which protrudes from the distal end face 41f of the ferrule 41 in a state in which the block body 51 is fitted in and firmly fixed to a concave portion 41i of the ferrule 41, if the length L of the free end of the illumination fiber 14 can be specified.

Note that the invention described in the aforementioned embodiments is not limited to the embodiments and modifications, and besides them, various modifications can be carried out within the range without departing from the gist thereof in the practical stage. Furthermore, the above-described embodiments include inventions in various stages, and various inventions can be extracted by proper combinations in a plurality of components that are disclosed.

For example, even if some components are deleted from all the components shown in the embodiments, if the problem that is mentioned can be solved, and the effect that is mentioned is obtained, the configuration from which the components are deleted can be extracted as the invention.

The present application is filed claiming the priority of Japanese Patent Application No. 2012-145926 filed in Japan on June 28, 2012, and the above described is incorporated in the description, claims and drawings of Japanese Patent Application No. 2012-145926.

## Claims

**1.** A scanning endoscope, comprising:

an optical fiber that guides a light with which a living body is illuminated;
a holding member having an insertion hole through which the optical fiber is inserted, in which the optical fiber is provided so as to extend from a distal end by a predetermined length and a concave portion that communicates with the insertion hole is formed at the distal end;
a drive portion that is provided in the holding member, and causes a free end of the optical fiber that extends from the distal end of the holding member to scan; and
an adhesive portion that is coated on or filled in the concave portion to firmly fix the optical fiber and the holding member to each other, and has a plane which is flush with the distal end of the holding member formed thereon.

**2.** The scanning endoscope according to claim 1,
wherein the concave portion is in a conical shape that is formed to open on the distal end of the holding member, and to be narrowed toward a proximal end side.

**3.** The scanning endoscope according to claim 1,
wherein the concave portion is in a pyramidal shape that is formed to open on the distal end of the holding member and to be narrowed toward a proximal end side.

**4.** The scanning endoscope according to any one of claims 1 to 3,
wherein the holding member is in a prismatic shape.

**5.** The scanning endoscope according to any one of claims 1 to 3,
wherein the holding member is in a columnar shape.

**6.** A production method of a scanning endoscope,
the scanning endoscope comprising:

an optical fiber that guides a light with which a living body is illuminated,
a holding member having an insertion hole through which the optical fiber is inserted, in which the optical fiber is provided so as to extend from a distal end by a predetermined length and a concave portion that communicates with the insertion hole is formed at the distal end,
a drive portion that is provided in the holding member, and causes a free end of the optical fiber that extends from the distal end of the holding member to scan, and
an adhesive portion that is coated on or filled in the concave portion to firmly fix the optical fiber and the holding member to each other, and has a plane which is flush with the distal end of the holding member formed thereon,
the production method comprising:

introducing the optical fiber into the insertion hole of the holding member;
extending the optical fiber to have a predetermined length from the distal end of the holding member;
coating or filling the concave portion with an adhesive;
scraping off an excess of the adhesive in accordance with a distal end face of the holding member to form a plane; and
curing the adhesive to form the adhesive portion.

**7.** The production method of a scanning endoscope according to claim 6,
wherein the adhesive is a thermosetting adhesive, and
when sinking by thermal curing of the adhesive occurs, coating or filling of the adhesive and thermal curing treatment are repeatedly performed until a surface portion of the adhesive portion becomes a plane which is flush with the distal end face of the holding member.

FIG.1

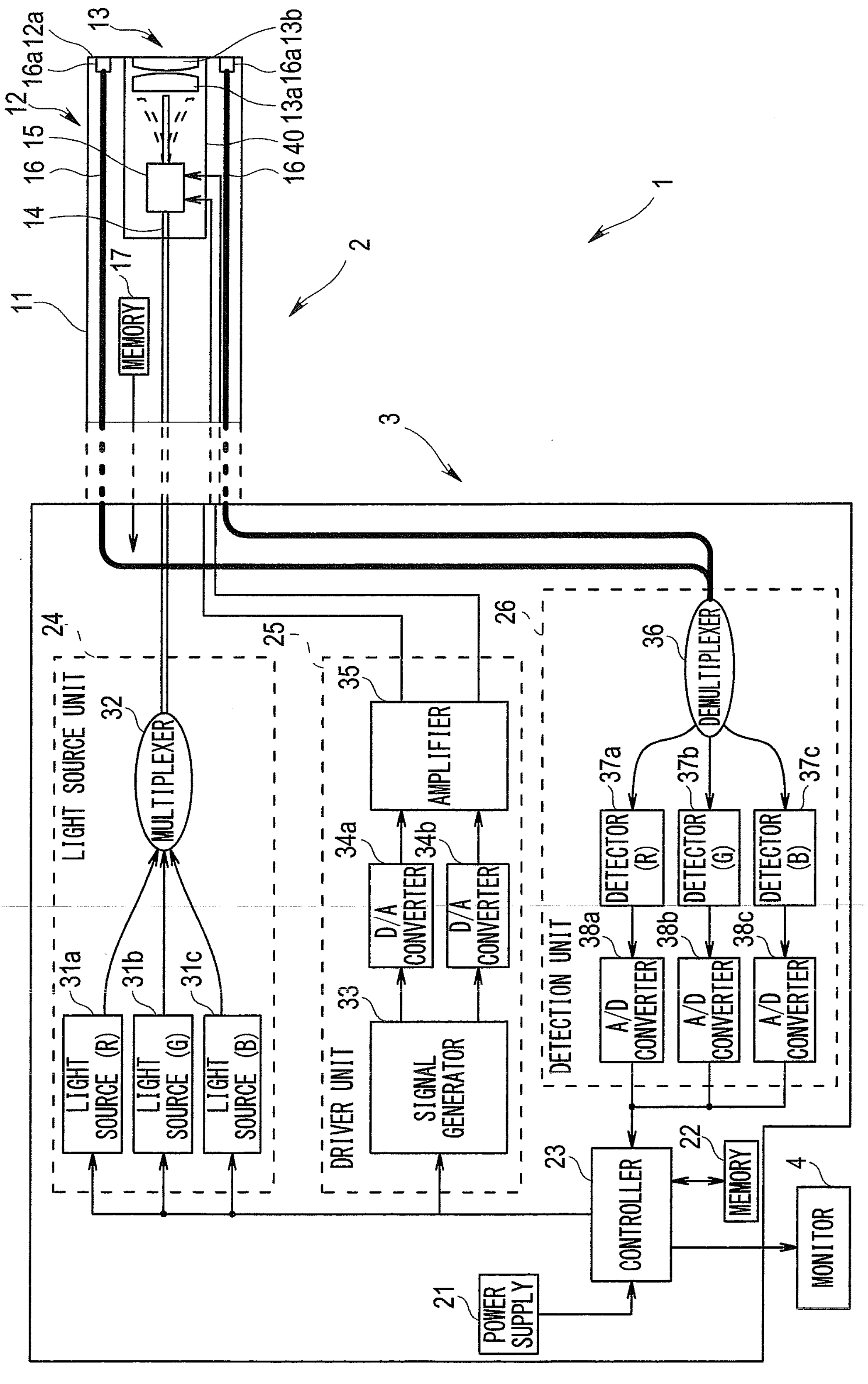

LIGHT SOURCE UNIT
LIGHT SOURCE (R)
LIGHT SOURCE (G)
LIGHT SOURCE (B)
MULTIPLEXER
DRIVER UNIT
SIGNAL GENERATOR
D/A CONVERTER
D/A CONVERTER
AMPLIFIER
DETECTION UNIT
DEMULTIPLEXER
DETECTOR (R)
DETECTOR (G)
DETECTOR (B)
A/D CONVERTER
A/D CONVERTER
A/D CONVERTER
CONTROLLER
MEMORY
MEMORY
MONITOR
POWER SUPPLY
1
2
3
4
11
12
12a
13
13a
13b
14
15
16
16a
17
21
22
23
24
25
26
31a
31b
31c
32
33
34a
34b
35
36
37a
37b
37c
38a
38b
38c
40

# FIG.2

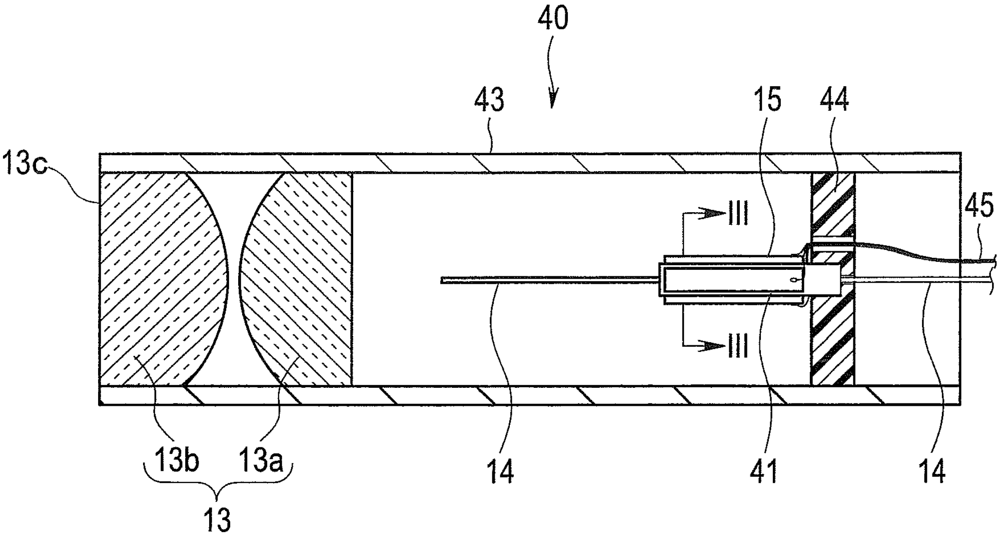
40
43
15
44
13c
45
III
III
13b
13a
13
14
41
14

# FIG.3

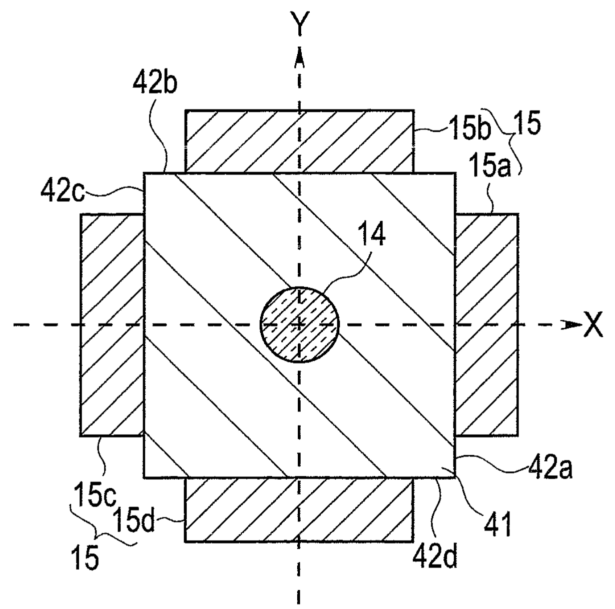
Y
42b
15b
15
15a
42c
14
X
42a
15c
15d
15
42d
41

FIG.4

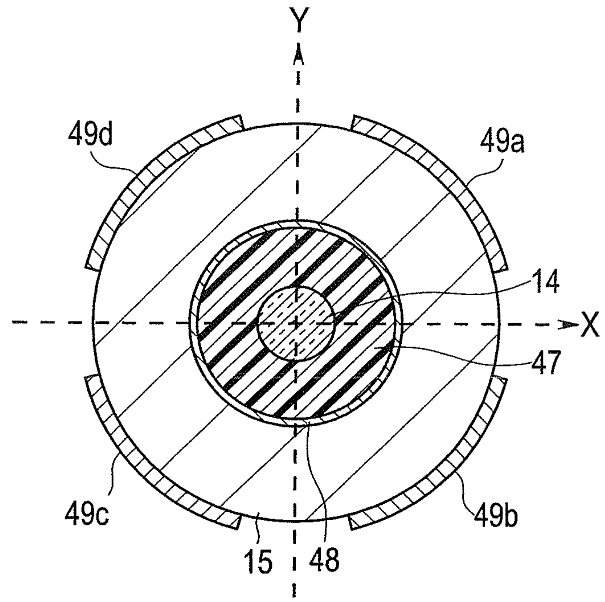


FIG.6

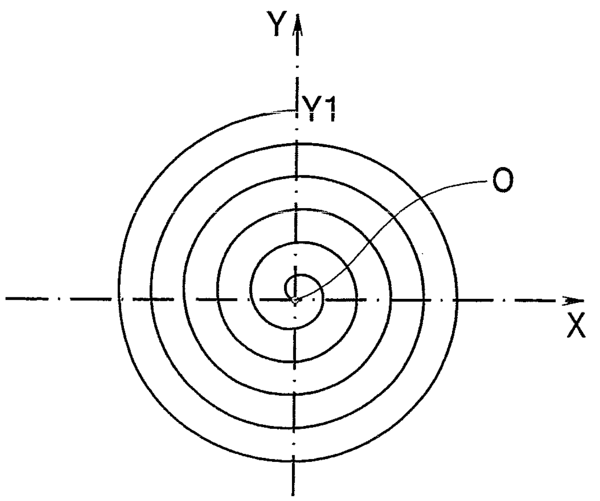

FIG.5

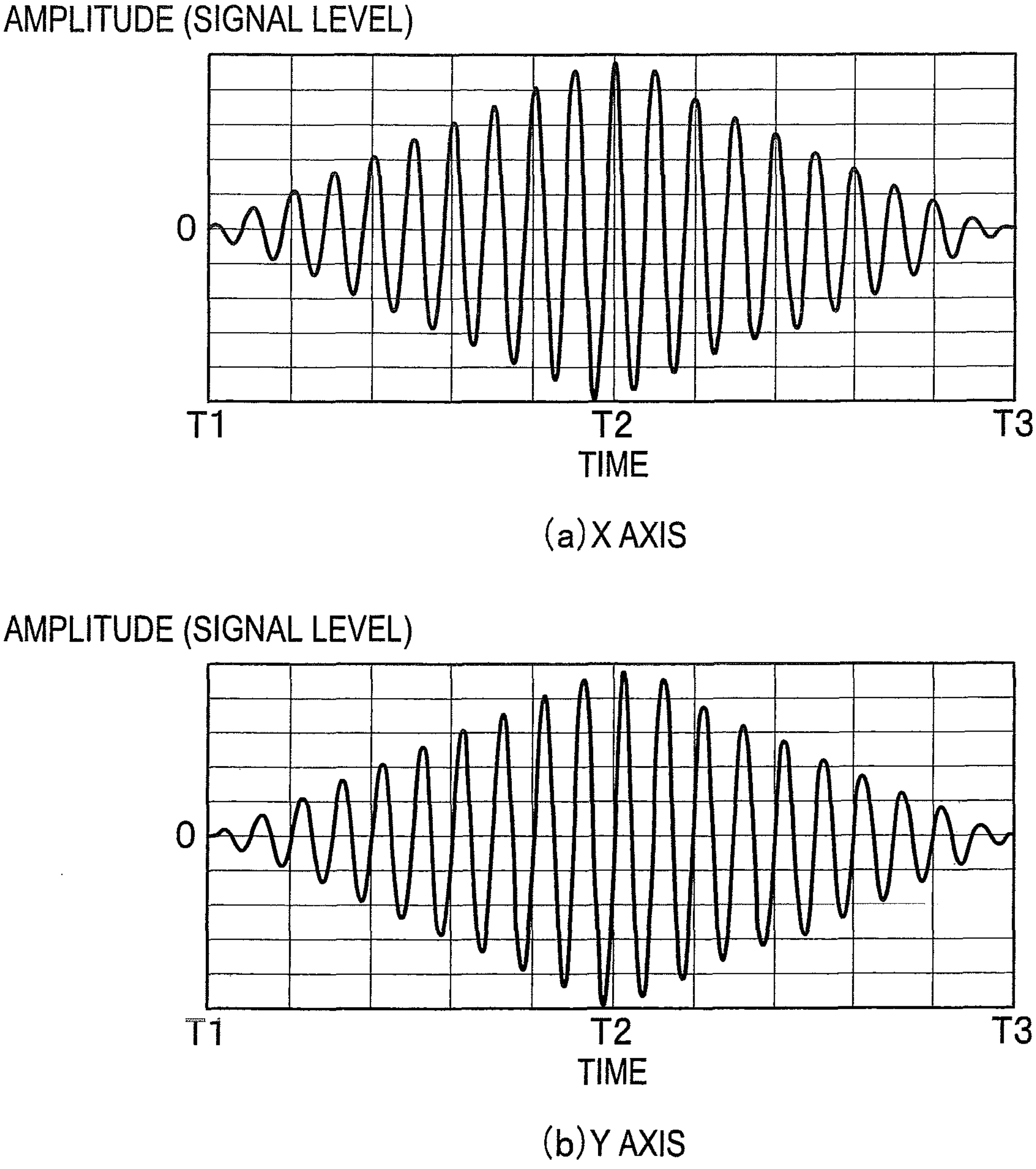
AMPLITUDE (SIGNAL LEVEL)
0
T1
T2
T3
TIME
(a) X AXIS
AMPLITUDE (SIGNAL LEVEL)
0
T1
T2
T3
TIME
(b) Y AXIS

# FIG.7

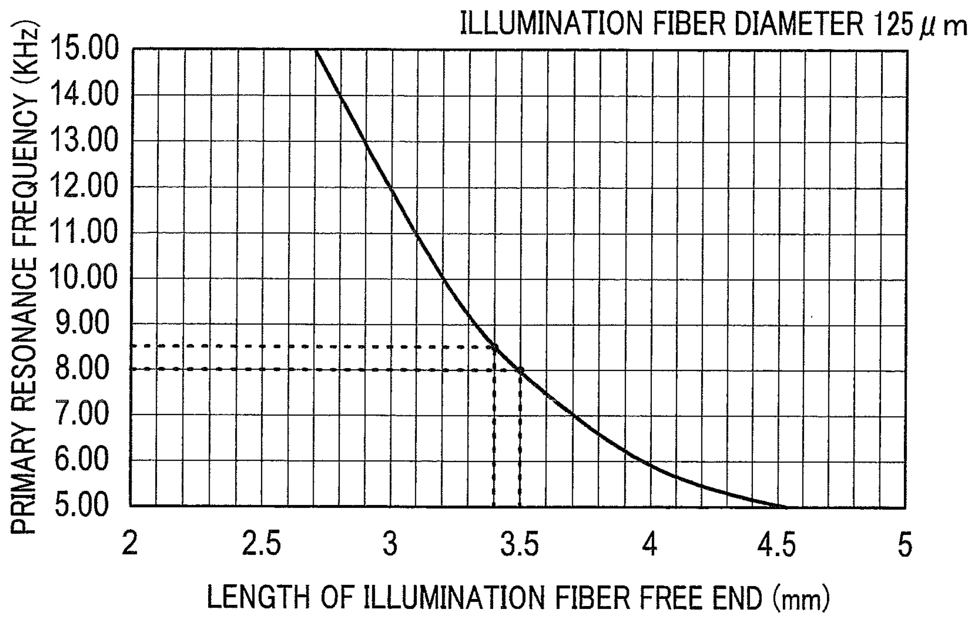
ILLUMINATION FIBER DIAMETER 125 μm
PRIMARY RESONANCE FREQUENCY (KHz)
15.00
14.00
13.00
12.00
11.00
10.00
9.00
8.00
7.00
6.00
5.00
2
2.5
3
3.5
4
4.5
5
LENGTH OF ILLUMINATION FIBER FREE END (mm)

# FIG.8

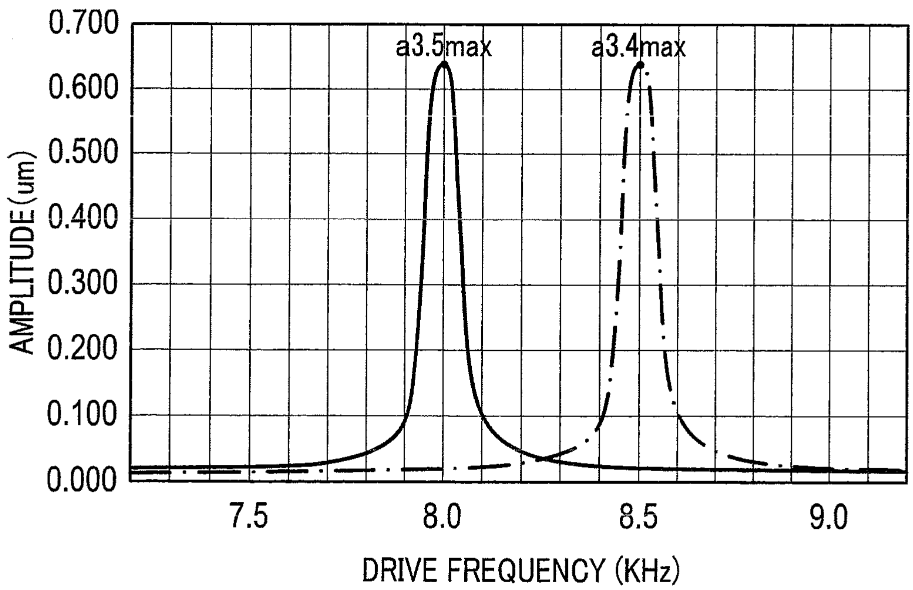
AMPLITUDE (um)
0.700
0.600
0.500
0.400
0.300
0.200
0.100
0.000
a3.5max
a3.4max
7.5
8.0
8.5
9.0
DRIVE FREQUENCY (KHz)

# FIG.9

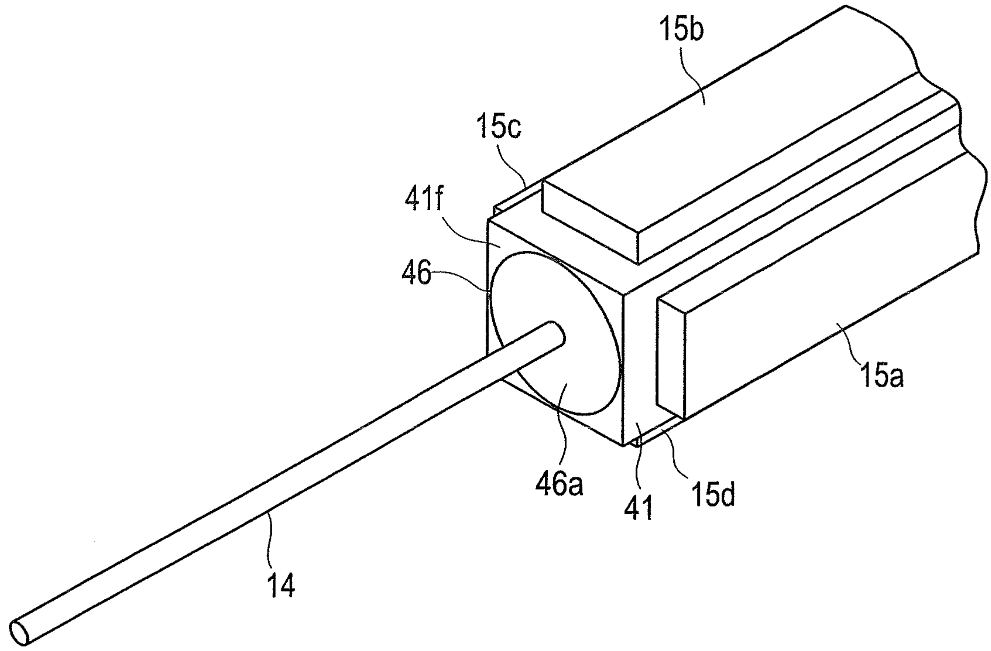


# FIG.10

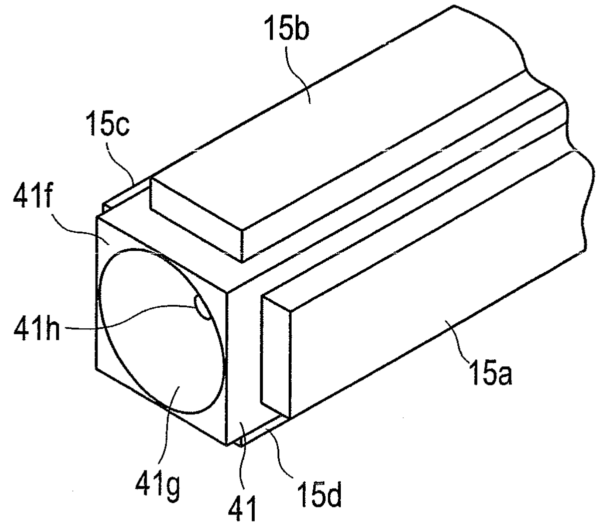

FIG.11

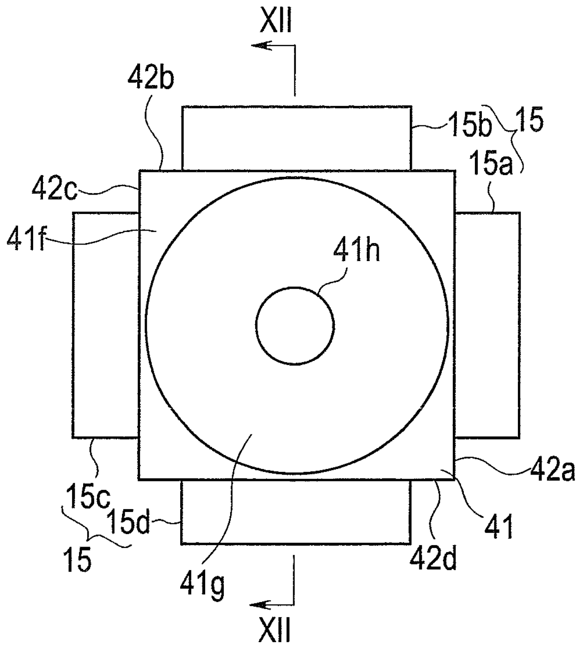
XII
42b
15b
15
15a
42c
41f
41h
42a
15c
15d
41
15
42d
41g
XII

FIG.12

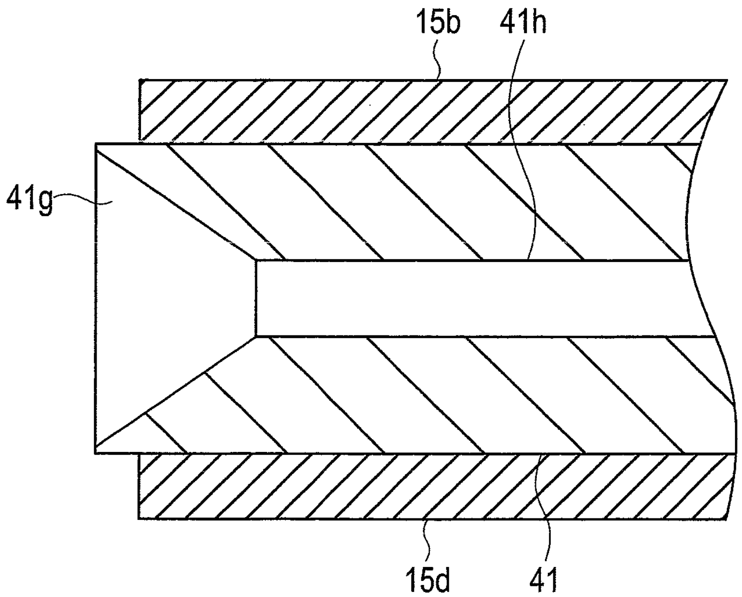
15b
41h
41g
15d
41

# FIG.13

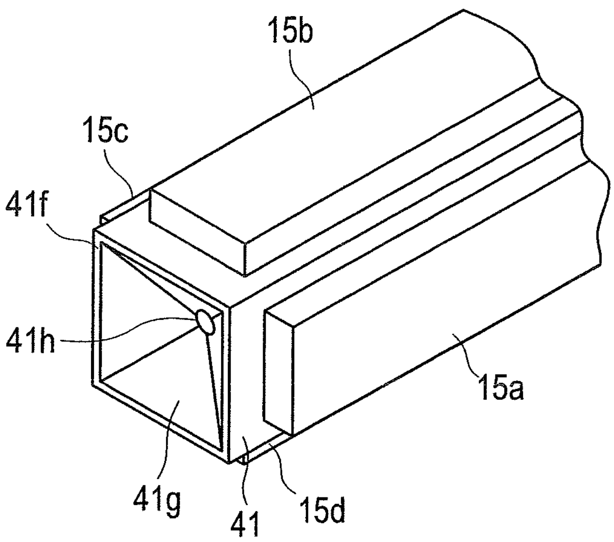
15b
15c
41f
41h
15a
41g
41
15d

# FIG.14

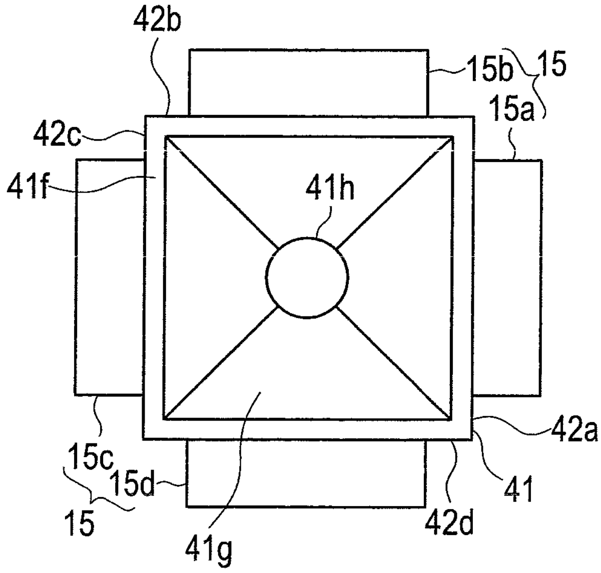
42b
15b
15
15a
42c
41f
41h
42a
15c
15d
41
15
42d
41g

FIG.15

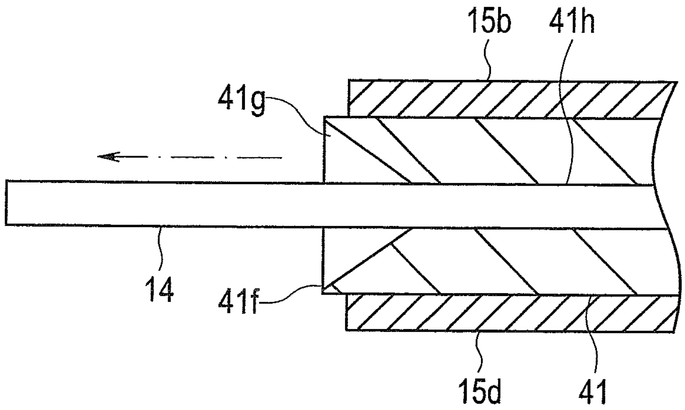


FIG.16

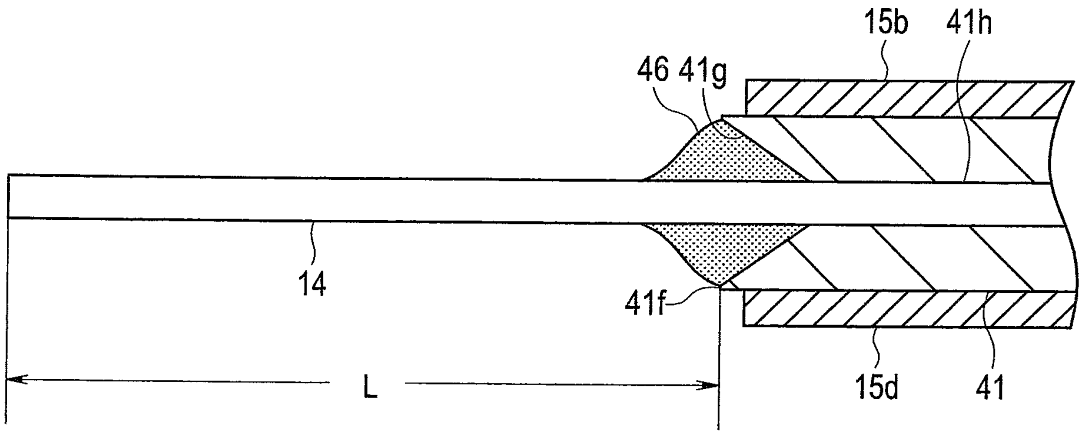


FIG.17

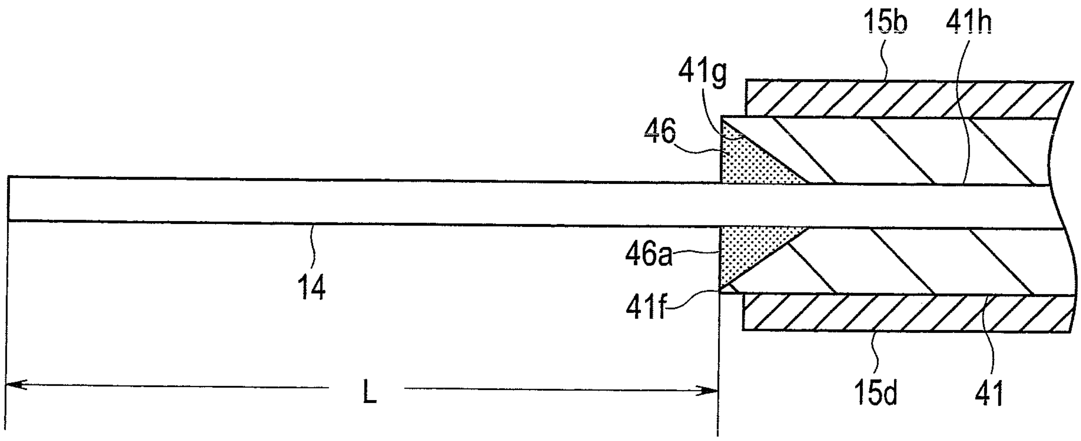

FIG.18

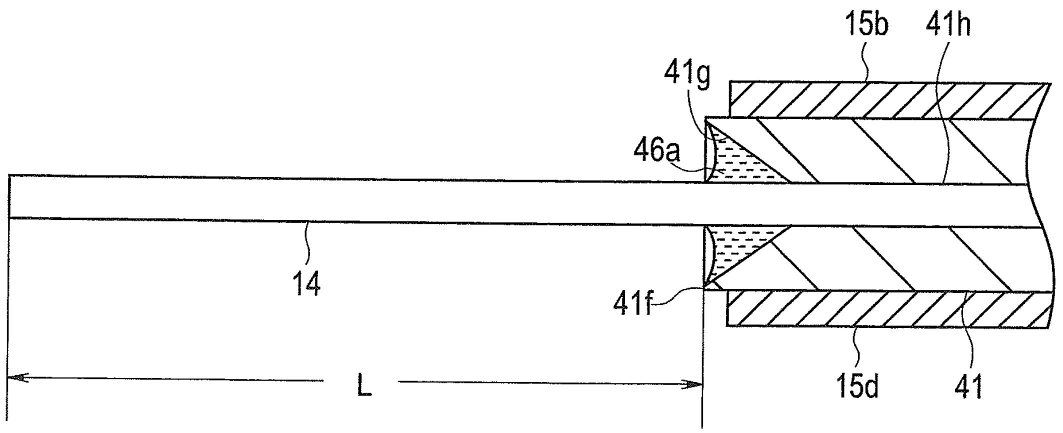


FIG.19

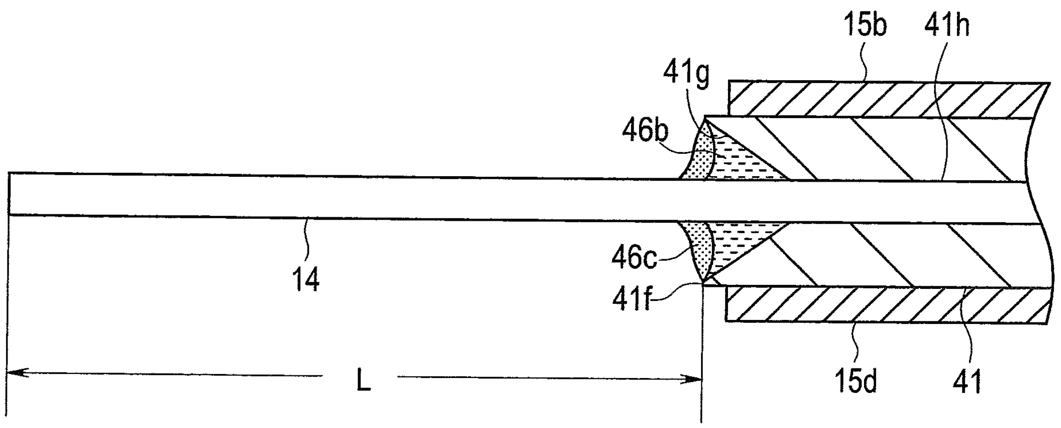


FIG.20

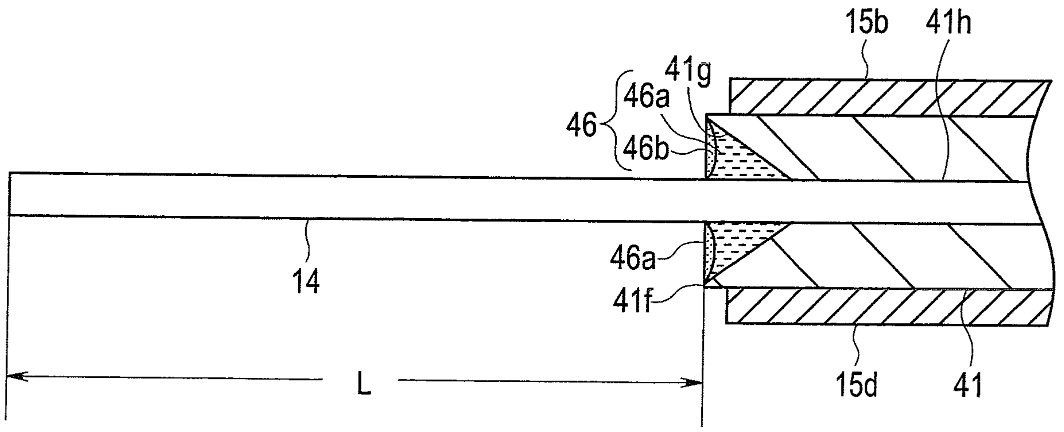

# FIG.21

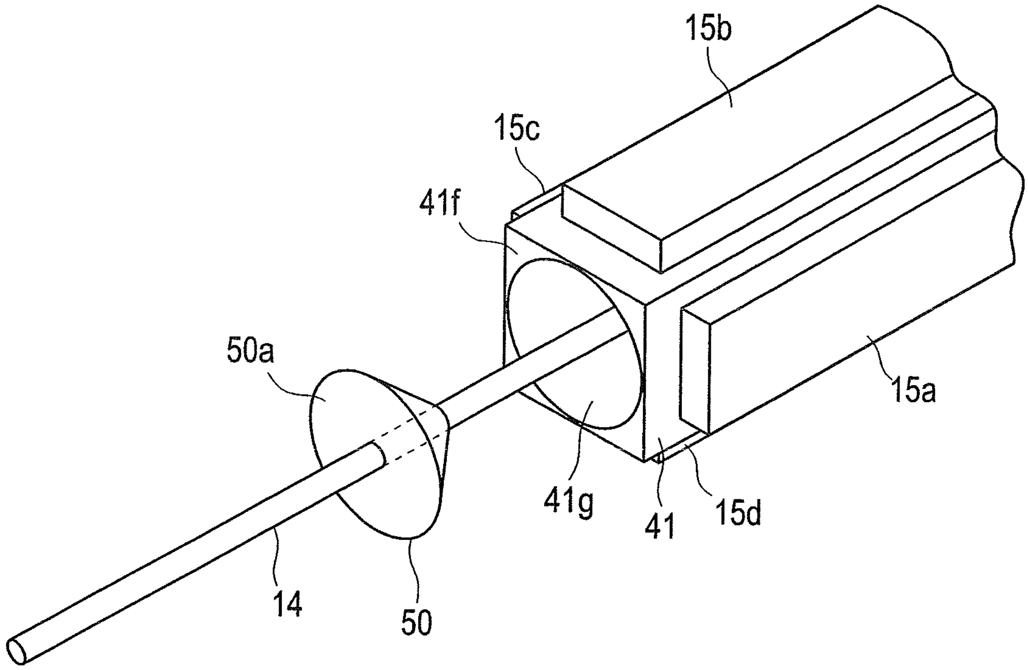
15b
15c
41f
50a
15a
41g
41
15d
14
50

# FIG.22

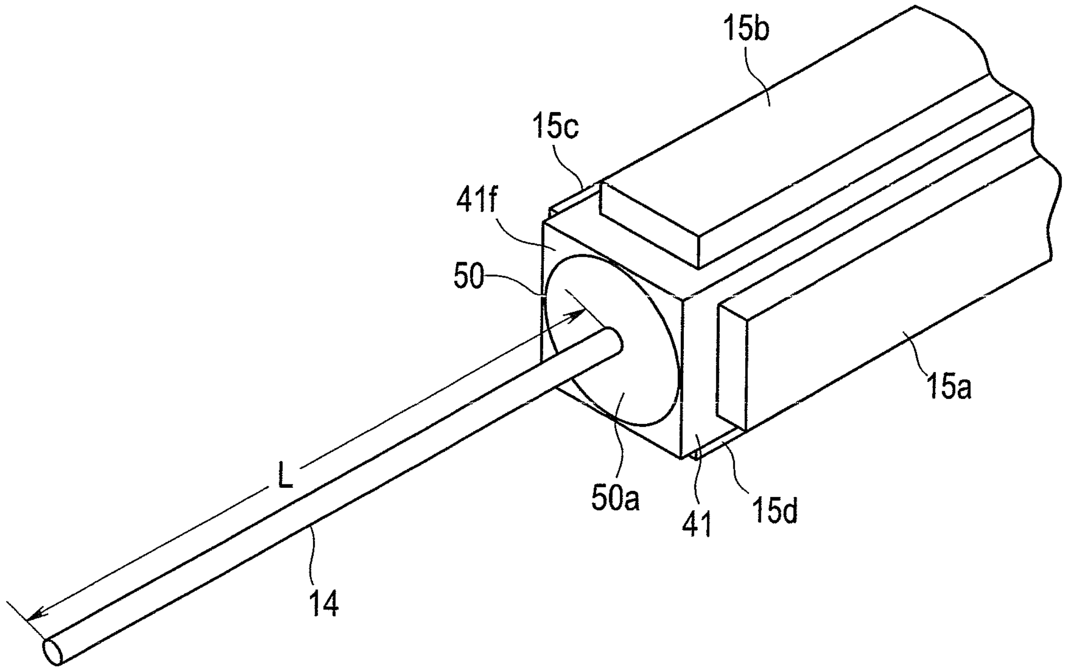
15b
15c
41f
50
15a
L
50a
41
15d
14

# FIG.23

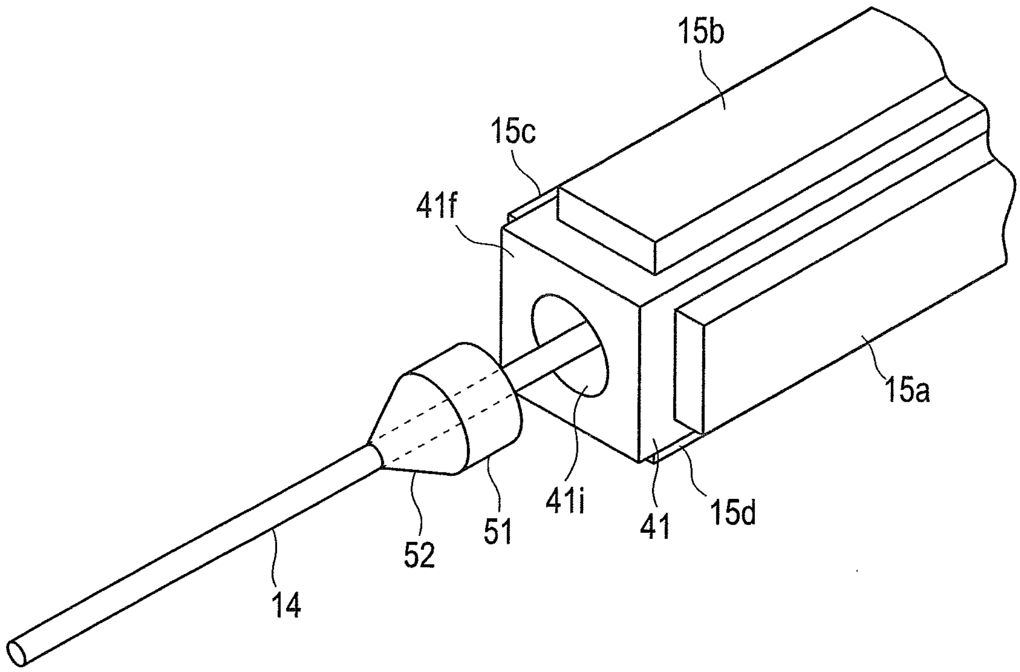


# FIG.24

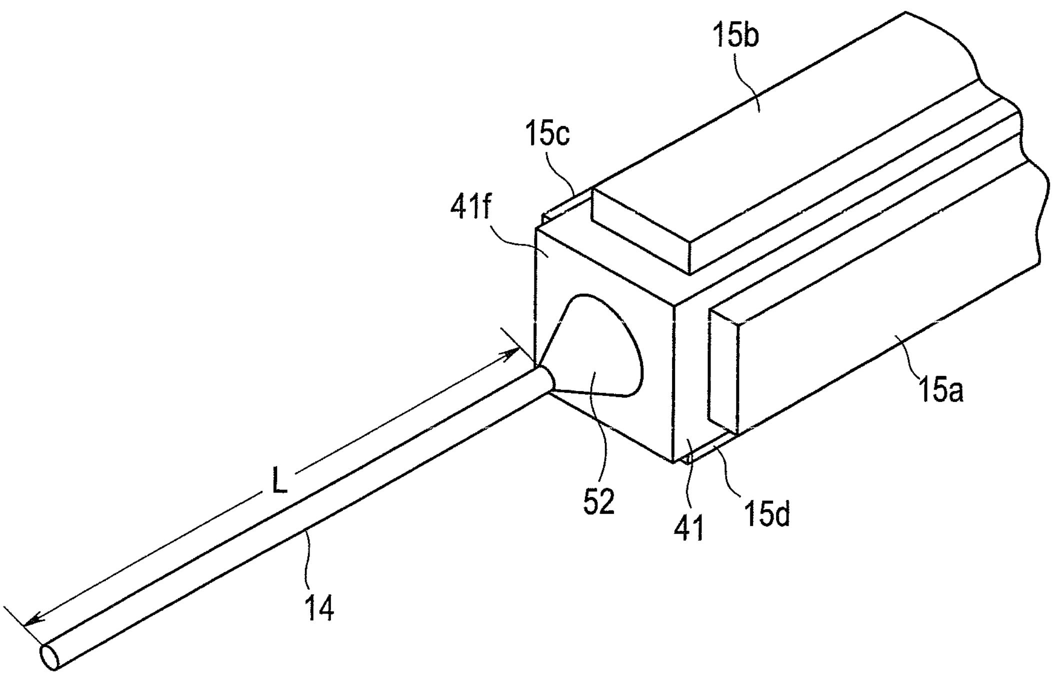

## INTERNATIONAL SEARCH REPORT

International application No.
PCT/JP2013/059241

A. CLASSIFICATION OF SUBJECT MATTER
*A61B1/00*(2006.01)i, *G02B23/26*(2006.01)i

According to International Patent Classification (IPC) or to both national classification and IPC

B. FIELDS SEARCHED

Minimum documentation searched (classification system followed by classification symbols)
A61B1/00-1/32, G02B23/24-23/26, G02B26/10

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2010-513949 A (UNIVERSITY OF WASHINGTON), 30 April 2010 (30.04.2010), entire text; fig. 1 to 9 & US 2008/0144998 A1 & US 2009/0103882 A1 & EP 2092388 A1 & WO 2008/076149 A1 & WO 2010/048234 A2 | 1-7 |
| A | JP 2001-174744 A (Olympus Optical Co., Ltd.), 29 June 2001 (29.06.2001), entire text; fig. 1 to 54 & US 7129472 B1 & EP 1142529 A1 & WO 2001/024686 A1 | 1-7 |

☐ Further documents are listed in the continuation of Box C.　☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 10 June, 2013 (10.06.13) | 18 June, 2013 (18.06.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2010513949 A
- JP 2012145926 A